# EUROPEAN PATENT APPLICATION

(11) **EP 4 567 729 A1**
(43) Date of publication of application: **11.06.2025**
(21) Application number: 23215277.7
(22) Date of filing: 08.12.2023
(51) Int. Cl.: G06T 7/10, G06T 11/60, G16H 50/00

(54) **COMPUTER-IMPLEMENTED METHOD FOR ENHANCED VISUALIZATION OF SURROUNDING TISSUE FEATURES OF A PATIENT'S ORGAN**

(71) Applicant: Inheart, 33600 Pessac (FR)
(72) Inventor: Soré, Bruno, 33600 PESSAC (FR); Juhoor, Mehdi, 33600 PESSAC (FR); Pannetier, Vincent, 33600 PESSAC (FR); Peyrat, Jean-Marc, 33600 PESSAC (FR)
(74) Representative: Aquinov

(57) **Abstract**

The invention concerns a computer-implemented method for the visualization of surrounding tissue features located out of a surface of a tissue of a patient's organ, the method comprising the following steps : receiving a 3D model comprising a mesh representing a surface of said tissue; receiving at least one image representing said tissue and at least one surrounding tissue feature located out of said surface; segmenting, in said image, at least one region of interest corresponding to said surrounding tissue feature and being formed by a plurality of first sub-parts; extracting, for each first sub-part, at least one value of a predetermined local characteristic; associating each sub-part to at least one mesh element of said mesh, based on said first sub-part and mesh element's positions; wherein said associated mesh elements form together a sub-mesh; each mesh element being labelled with the value of the predetermined local characteristic of its associated first sub-part.

## Description

The present invention relates to the field of computer assisted medical imaging and visualization technologies. More particularly, the invention relates to a computer-implemented method for the accurate and detailed visualization of surrounding tissue features located out of the surface of a tissue of a patient's organ.

In the realm of medical imaging and, more precisely, in the visualization of organ tissues and surrounding anatomical structures, practitioners have been continuously confronted with several challenges, particularly in the context of acquiring detailed, accurate, and comprehensive visual data. Traditional imaging methodologies, such as MRI, CT scans, and ultrasound, have undeniably broadened the medical capabilities to perceive and evaluate internal bodily structures. However, the discernment of adjacent or surrounding tissue features, especially those existing outside or inside of a given tissue surface, remains suboptimal.

It is crucial to accurately understand how an organ tissue and its nearby tissues relate to each other in space. This understanding aids medical experts in many ways. For example, in cancer care, knowing exactly where a tumor ends and how close it is to important structures helps in planning surgeries and predicting the disease's course. In heart care, seeing the heart tissue and nearby fat and blood vessels clearly helps in diagnosing issues and planning treatments. Combining a 3D model of an organ tissue with medical images provides a clearer view, making it easier for medical experts to understand the situation and make informed decisions. This enhanced view helps improve diagnosis, treatment planning, and patient outcomes, making it a necessary step forward in medical practice.

In contemporary computer-assisted medical imaging technologies, 3D models of organs and tissues have been widely adopted to facilitate an immersive and intricate understanding of anatomical structures. Nevertheless, these models often focus predominantly on the organ or tissue itself, at times inadequately representing surrounding or adjacent tissue features. Such a limitation can hinder the accurate visualization and evaluation of the comprehensive anatomical landscape, thereby potentially affecting diagnostic and therapeutic decision-making processes.

Moreover, while these technologies provide static, often high-fidelity representations of internal structures, they can fall short in delivering a seamless integration of real-time or static imaging data with pre-existing 3D models. The task of aligning and associating 2D image data, especially related to surrounding tissue features, with the 3D model's mesh elements often demands manual intervention and/or complex time-consuming computational methodologies.

Current methods may lack the precision or automation necessary to accurately identify, segment, and integrate relevant data from images, particularly when it comes to discerning between the tissue of interest and adjacent features based on specific local characteristics, such as intensity and/or relative distance. Conventional approaches might employ rudimentary thresholding techniques and simplistic region-of-interest (Rol) extraction methodologies, which could potentially result in the incorporation of inaccurate, insufficient, or mis-representative data into the 3D model.

Consequently, a need arises for a sophisticated, computer-assisted methodology capable of seamlessly assimilating detailed 2D image data into 3D organ or tissue models, ensuring that both the principal tissue and its surrounding features are accurately and comprehensively represented.

The purpose of the present invention is to overcome the limitations of the prior art solutions, particularly those outlined here above, by providing a computer-implemented method for the visualization of surrounding tissue features located out of a surface of a tissue of a patient's organ, the method comprising:
a. step of receiving a 3D model of a tissue of said patient's organ, said 3D model comprising a mesh representing a surface of said tissue of said patient's organ;
b. step of receiving at least one image representing said tissue and at least one surrounding tissue feature located out of said surface of said tissue;
c. step of segmenting, in said image, at least one region of interest corresponding to said surrounding tissue feature, said region of interest being formed by a plurality of first sub-parts;
d. step of extracting, from said image and for each first sub-part of said region of interest, at least one value of a predetermined local characteristic of said first sub-part of said region of interest;
e. step of associating each sub-part of said region of interest to at least one mesh element of said mesh of the 3D model, based on said first sub-part and mesh element's positions; wherein said associated mesh elements form together a sub-mesh of said mesh; each mesh element of said sub-mesh being labelled with the value of the predetermined local characteristic of its associated first sub-part of said region of interest.

To this end, the devised methodology provides a robust solution for comprehensively visualizing not only the primary tissue of a patient's organ but also adeptly detailing surrounding tissue features, particularly those located at the vicinity of the tissue's boundaries, in particular inside or outside the tissue surface. The incorporation of a meticulously constructed 3D model, featuring a representative mesh, serves to provide a substantive structural foundation upon which additional image data can be overlaid and integrated. The process of receiving and integrating at least one image that portrays the tissue and adjacent features offers the ability to add real-world, patient-specific data to the structural 3D model.

Through the strategic segmentation of regions of interest within the image, which correspond to the surrounding tissue features, and the subsequent extraction of predefined local characteristics from these segmented regions, the method ensures that the visualized data is not only accurate but also rich in detail, providing nuanced insights into the specificities of the surrounding tissue features. This segmentation and extraction process thereby elevates the accuracy and specificity of the resultant visualization, ensuring that the adjacent features are precisely and informatively represented.

Furthermore, by associating each sub-part of the segmented region of interest with corresponding elements within the 3D model's mesh and forming a sub-mesh which is labeled with specific local characteristic values, the method delivers a seamless integration of 2D and 3D data. This not only enhances the fidelity and depth of the resultant visualization but also ensures that the spatial and characteristic relationships between the primary tissue and surrounding features are accurately maintained and represented within the model.

Thus, this methodology ensures a high-fidelity and detailed representation of both the primary tissue and adjacent features, thereby providing a more comprehensive visualization for medical practitioners. Secondly, it mitigates the potential for manual data entry errors and enhances efficiency by automating the process of image data integration with 3D models. Consequently, this could potentially streamline diagnostic and therapeutic decision-making processes by providing a more accurate and detailed visual insight into the anatomical structures of interest. This amalgamation of 2D imaging data with 3D models via an automated and precise method represents a significant advancement, potentially offering improved outcomes in diagnostic and therapeutic interventions.

If desired, the segmentation step can be executed employing various image processing techniques and algorithms to identify regions of interest within the image data. For example, traditional thresholding techniques can be used to differentiate the tissue features based on intensity values, while more advanced machine learning algorithms, such as convolutional neural networks, can be trained to identify and segment the regions of interest with higher accuracy and efficiency, especially in the presence of noise or when the features are not distinctly separable through traditional methods.

Advantageously, the extraction of predetermined local characteristics can leverage various image analysis methodologies. For instance, texture analysis could be used to extract features related to the spatial distribution of intensities in the segmented regions, while shape descriptors could be employed to capture the geometrical and topological properties of the segmented features, providing additional insights into the nature and potential pathology of the surrounding tissue features.

If desired, the step of associating each sub-part of the segmented region to mesh elements of the 3D model can be achieved through various registration and mapping techniques. For instance, rigid, affine, or non-rigid registration methodologies can be utilized to accurately align the 2D image data with the 3D model, ensuring that the spatial relationships between the features are preserved. Alternatively, geometric hashing or point cloud alignment techniques could be explored to establish an accurate mapping between the segmented image features and the mesh elements of the 3D model, ensuring precise localization and representation of the surrounding tissue features. Alternatively again, said associating step might comprise, for each subpart of said region of interest, the identification of the mesh element or of the group of mesh elements of said mesh of the 3D model which is the closest to said sub-part, said identified mesh element or group of mesh elements being then associated to said sub-part.

In an alternative embodiment, the creation of the 3D model may leverage different imaging modalities or synthetic data, depending on the specific application and requirements. For example, ultrasound or optical coherence tomography data could be utilized to construct the 3D model, especially in applications where real-time imaging is beneficial, such as during surgical interventions.

In the context of the present specification, unless expressly provided otherwise, a "computer" may refer, but is not limited to, a desktop computer, a laptop computer, a tablet computer, a piece of testing equipment, a network appliance, a controller, a digital signal processor, a computational engine within an appliance, a consumer-electronic device, a portable computing device, and/or another electronic device, and/or any combination thereof appropriate to the relevant task at hand. Moreover, the method steps might be all executed on a same device. As a variant, the method steps might be executed on several devices, connected by wires or by a wireless connection.

In the context of the present specification, a "3D model of a tissue of an organ" might comprise one or more structured mesh, each comprising a plurality of vertices connected with each other to define a plurality of faces and/or volumes which approximate internal and/or external surfaces and/or volumes of a tissue of the organ. A 3D model might be a triangle mesh or a quadrilateral mesh, or more generally a polygonal mesh, which comprises a plurality of vertices, with edges connecting pairs of vertices and/or polygonal faces connecting a closed set of vertices. As a variant, a 3D model might be a tetrahedral mesh or a hexahedral mesh or a prism mesh, or more generally a volume mesh, which comprises a plurality of vertices, with polyhedrons connecting a closed set of polygons defined by closed set of vertices. Preferably, each vertex and/or edge and/or polygon and/or polyhedron might be labelled as being a part of a specific sub-part of the heart. A 3D model might be stored as a list of vertices each associated with spatial coordinates and with a set of connections to other vertices, or as a list of vertices associated with spatial coordinates and a list of polygons or faces each defined by a subset of vertices contained in said list of vertices, being understood that any other suitable method of storage might be used in the context of the present specification. A mesh element might be then a vertex of the 3D model or a face or a volume defined by connected vertices of the 3D model.

In the context of the present specification, said "3D model" might be (or might be computed from) one of, or a combination of two or more of : an electrophysiological map, resulting from an electrocardiogram (ECG) and/or from an invasive mapping, an anatomical and/or a functional map, resulting from a computed tomography(CT)-scan, a spectral computed tomography(SCT)-scan, a positron emission tomography(PET)-scan, a single photon emission computed tomography(SPECT)-scan, a photon-counting computed tomography(PCCT)-scan or a magnetic resonance imaging(MRI)-scan, and an electro-anatomical map, resulting from an invasive mapping.

Advantageously, the disclosed method can be implemented in various medical contexts to enhance the visualization of organic tissues and their surrounding features, thereby facilitating more informed medical decision-making. For example, the method can be employed in cardiology to augment the visualization of a 3D model of cardiac tissues such as the epicardium, endocardium and/or myocardium of the left atrium; as well as surrounding structures such as fat clusters, blood vessels or arrhythmia scars. This approach enables an enriched visual representation that can prove crucial for the accurate diagnosis and treatment planning of cardiac conditions. Alternatively, a similar application of the method can be envisaged in the field of neurology, where the augmented visualization of a 3D model of brain tissue and surrounding structures can provide valuable insights for the management of neurological disorders.

Advantageously, the method articulated can be implemented in various medical contexts to enhance the visualization of organ tissues and their surrounding features, facilitating more informed medical decision-making. For instance, the method can be employed in cardiology to augment the visualization of a 3D model of heart tissues such as the endocardium, the epicardium, the pericardium, and the myocardium; as well as the nearby structures such as fat pads, blood vessels or arrythmia scars.

In neurosurgery, where accurate visualization of brain tissues and adjacent structures is paramount, the method can utilize a 3D model derived from pre-operative MRI scans. This model, which comprises a mesh representing the brain tissue surface, can be enriched with subsequent CT or further MRI scans that illuminate surrounding tissue features, such as tumors, blood vessels, or other pathological entities.

In the context of the present specification, unless expressly provided otherwise, "an image representing a tissue" refers to a graphical representation of biological tissues obtained through various imaging modalities, such as MRI, CT, ultrasound, or others. These images might be in 2D, 3D, or 4D (3D over time or 3D plus another parameter like blood flow velocity) formats and are composed of fundamental imaging units: pixels for 2D images and voxels for 3D/4D images. Each pixel or voxel holds specific information about a small, discrete portion of the tissue, such as intensity or color, which is used to visualize the structure and characteristics of the examined tissue in the respective dimensional space.

In this context, the Region of Interest (Rol) corresponding to surrounding features in medical imaging denotes a selected segment of the overall image that holds particular significance or relevance for the ongoing analysis or diagnostic process. For the context of visualizing surrounding tissue features located out of a surface of a tissue of a patient's organ, the region of interest could correspond to areas either inside or outside the volume defined by the surface of the tissue, depending upon the specific medical case or procedure. In cardiology, especially while examining the atrium, fat pads or an arrhythmia scar could be deemed as region of interest due to their relevance in understanding cardiac functionality or diagnosing particular conditions. The Region of interest allows healthcare professionals to focus their analysis and visual representation on specific structures or abnormalities (like scars or fat pads) that might impact the cardiac rhythm or other functionalities.

In the context of the present specification, unless expressly provided otherwise, "sub-part", typically refers to a smaller, defined portion or segment of the region of interest. It can be a fundamental imaging unit, meaning a single pixel or voxel, or alternatively, it may encompass a group of pixels/voxels. This segmentation into sub-parts allows for a detailed, nuanced analysis and processing of the region of interest, enabling the extraction of localized characteristics and facilitating the subsequent steps of the method, such as associating sub-parts with mesh elements in a 3D model. This kind of granularity is especially vital when dealing with heterogenous regions of interest, wherein different sub-parts may exhibit varied local characteristics pertinent to diagnosis or analysis.

In the context of the present specification, unless expressly provided otherwise, "a predetermined local characteristic of a sub-part of a region of interest" might be a physiological, a physical or a geometrical characteristic of a surrounding tissue feature whose location corresponds to said sub-part. A physiological characteristic might be a tissue density. A physical or a geometrical feature might be a tissue thickness, such as the thickness of a structure composed of adipose tissues surrounding the heart, or a value indicating a distance between said organ and said surrounding feature. It is understood that any other suitable local characteristic of the surrounding feature, which can help practitioner to, directly, or indirectly and partly, or fully, diagnose a condition related to said feature or prepare an intervention on said feature, might be used in the context of the present specification.

In one embodiment of the invention, the segmentation step comprises the selection of a plurality of fundamental imaging units and/or a plurality of groups of fundamental imaging units within the image which match, individually or collectively, at least one predetermined criterion, whereby this selection forms said region of interest. For instance, said segmentation step comprises the identification and selection of pixels/voxels based on their intensity being either above or below a particular threshold and/or their spatial position relative to the surface of the tissue being within certain predefined proximity limits.

The position criterion might stipulate that the distance to the tissue surface must be below a certain threshold or within a specified range and may also include considerations for selecting connected pixels to ensure the continuity and logical coherence of the identified region of interest, thereby aligning with and meticulously highlighting the desired surrounding tissue features for subsequent analytical and visualization processes.

This ensures a specific, targeted focus on surrounding tissue features that not only are proximal to the primary tissue but also exhibit intensity values indicative of particular characteristics or pathologies. Consequently, this enhanced segmentation method filters and isolates relevant sub-parts of the image, based on their spatial and intensity relationships with the primary tissue, providing a more nuanced and targeted visualization, particularly useful in identifying and visualizing specific features or abnormalities that exist in close proximity to the primary tissue. This approach ensures that the resultant visualization is both clinically relevant and streamlined, reducing the potential for extraneous data to obfuscate the visualization and focusing the analytical perspective on critical regions adjacent to the tissue of interest.

To this end, the segmentation step, which selectively considers any sub-part of the image that is situated outside of the tissue, whose intensity falls below a predetermined threshold, and/or whose proximity to the tissue is within a specified limit, brings forth substantial advantages and impactful technical effects in the visualization of surrounding tissue features. Specifically, this precise approach in the segmentation process aids in homing in on potentially relevant or pathologically significant features that are closely abutting the main tissue, thus maintaining a critical focus on immediately adjacent structures or abnormalities that might have crucial implications for surgical planning or diagnostic assessment.

The utilization of an intensity threshold serves to filter and delineate features based on their inherent radiological properties, enabling the differentiation and identification of structures or features that may correspond to various tissue types or pathological states, thereby enhancing the specificity and relevance of the resultant visualization. This discernment is particularly advantageous in scenarios where nearby features might be of disparate clinical relevance, ensuring that the visualization prioritizes and highlights those features that are not only spatially relevant but also indicative of specific clinical or pathological states based on their intensity characteristics.

Alternatively or cumulatively, the imposition of a spatial threshold for the proximity of the surrounding features to the primary tissue further streamlines the visualization, safeguarding against the inclusion of extraneous or clinically irrelevant features that might be located further afield. This focused visualization, therefore, is poised to present clinicians with a clear, uncluttered, and contextually relevant view of the tissue and its immediate surroundings, thereby optimizing the clarity and utility of the visual information provided, which is pivotal during crucial medical decisions and interventions.

Thus, the strategic segmentation, underpinned by defined intensity and spatial thresholds, not only facilitates a focused, relevant, and clear visualization of the tissue and its adjacent features but also minimizes the risk of visual clutter and distraction from less relevant structures. This meticulous approach, therefore, is paramount in ensuring that the visualized data is maximally pertinent, clear, and actionable, thus supporting enhanced clinical decision-making and planning, especially in contexts where the precision, clarity, and relevance of visual data are critical. Consequently, this methodology represents a significant technical advancement in ensuring that 3D visualizations are not only detailed and accurate but also clinically pertinent and contextually optimized.

Advantageously, the predetermined intensity and distance thresholds utilized during segmentation could be dynamically adjustable, allowing clinicians to modify these parameters in real-time or during pre-visualization processing, thus adapting the visualization to varied clinical scenarios or requirements. For instance, in the visualization of pancreatic tissue and adjacent structures, adjusting the intensity threshold might allow for the selective visualization of adjacent vascular structures or potential tumor formations, providing clinicians with a flexible and adaptive visualization tool.

Advantageously, said segmentation step comprises the selection of any sub-part of said image which is located outside of said tissue, whose distance to said tissue is lower than a predetermined threshold. Preferably, said segmentation step might comprise the selection of any sub-part of said image which is located outside of said tissue, whose intensity is lower than a predetermined threshold and whose distance to said tissue is lower than a predetermined threshold

In this example when applied to an image of a heart atrium, the segmentation step might help to identify one or more regions of interest corresponding each to an adipose tissue surrounding the atrium's epicardium, namely "fat pad". For instance, said intensity predetermined threshold can be set to 10 Hounsfield Units and said distance predetermined threshold can be set to 1 cm.

In another example, said segmentation step comprises the selection of any sub-part of said image which is located between two surfaces of said tissue and whose intensity is greater than a predetermined threshold. In this example when applied to an image of a heart atrium, the segmentation step might help to identify one or more regions of interest corresponding each to a part of an arrhythmia scar in the atrium's myocardium. For instance, said intensity predetermined threshold can be set to 10 Hounsfield Units and said distance predetermined threshold can be set to 1 cm.

If desired, machine learning algorithms could be incorporated to augment the segmentation step, especially in scenarios where surrounding tissue features might exhibit variability in their intensity or spatial characteristics. Employing a trained model to recognize and segment these features, even in the context of varied or inconsistent radiological characteristics, ensures that the visualization is robust and reliable, even in the context of complex or varied clinical scenarios.

In a cumulative embodiment of the invention, the extraction step comprises, for a plurality of second sub-parts of said image corresponding to said surface of said tissue; determining a trajectory encompassing said second sub-part and at least one first sub-part of said region of interest; and wherein, for each first sub-part of said region of interest, said value of a predetermined local characteristic is computed depending on said trajectory which encompasses said first sub-part of said region of interest.

Advantageously, said extraction step might comprise a step of segmenting said image to identify a plurality of second sub-parts of said image which correspond to said surface of said tissue. Said segmentation might be implemented with any suitable image processing method, for instance intended to identify pixels or voxels which delimits a volume of a heart in said image, said pixels or voxels corresponding to a heart tissue as the epicardium or the endocardium.

Advantageously, the elaboration in the method where the extraction step involves determining a trajectory encompassing parts of the image corresponding to the surface of the tissue, as well as the surrounding tissue features, augments the precision and contextual relevance of the visualization process. By computing the value of a predetermined local characteristic based on the trajectory which encompasses both the tissue surface and the surrounding tissue features, the method enables a more informed, relationally accurate representation of the anatomical structures in question. This nuanced approach not only enhances the accuracy of visualizations but also potentially enriches the information that can be derived from the medical imaging, thereby facilitating more informed diagnostic or therapeutic decisions. The method's ability to relate the surface tissue characteristics with those of the surrounding tissue features through a defined trajectory significantly elevates the quality and the utility of the medical imaging output, making it a valuable asset in clinical settings.

Advantageously, by adhering to a predetermined constraint where each pixel of the region of interest traversed by a trajectory encompassing a pixel of the tissue's border is associated to said pixel of the tissue's border, the method allows for a structured and coherent association step. This specific arrangement ensures that the relationship between the surface of the tissue and the surrounding tissue features is accurately maintained and represented in the visualization. The deliberate association based on the traversal by trajectory provides a logical and precise framework, ensuring that the ensuing medical imaging output is both accurate and meaningful, thereby potentially aiding in more accurate diagnoses or treatment planning.

In a cumulative embodiment of the invention, each trajectory is a trajectory encompassing a second sub-part corresponding to said surface of said tissue and at least one first sub-part of said region of interest with a direction normal to said surface at the location of said second sub-part.

Advantageously, the delineation in the method where each trajectory is defined to have a direction normal to the tissue surface at the location of the associated tissue surface sub-part, further refines the relational accuracy between the tissue surface and surrounding tissue features. By ensuring that each trajectory extends normally from the tissue surface, a more accurate spatial relationship between the tissue and the surrounding tissue features is established. This approach minimizes any spatial distortion that might arise from arbitrary trajectory directions and ensures a true-to-anatomy visualization of the tissue and its surrounding features. This normal trajectory also streamlines the computational process by providing a straightforward geometric relationship, which could potentially lead to faster processing and more immediate visualization results, aiding in timely medical analysis and decision-making. The method's meticulous approach to defining the trajectory ensures a high degree of precision and reliability in the medical imaging output, which is essential in clinical settings for accurate diagnostics and effective treatment planning.

In a alternative or cumulative embodiment of the invention, said trajectories are determined with:
a). Dilating the plurality of second sub-parts to define a plurality of third sub-parts which englobes said region of interest;
b). Determining a plurality of trajectories each joining one second sub-part to one third sub-part.

Advantageously, the specified mechanism of determining trajectories in the method, through dilation of the second sub-parts to define a new set of third sub-parts which envelop the region of interest, and subsequently establishing trajectories between the second and third sub-parts, facilitates a more encompassing and accurate representation of the anatomical structures in question. This particular approach ensures that the trajectories accurately bridge the tissue surface and the surrounding tissue features, thereby enhancing the spatial accuracy and contextual relevance of the visualization. By methodically establishing a network of trajectories that accurately map the spatial relationships between the tissue and surrounding features, a more robust and detailed visualization is achieved. This, in turn, may lead to more informed diagnostic evaluations or therapeutic interventions, as the medical imaging output will provide a thorough and precise representation of the anatomical structures and their spatial interrelations. Through this meticulous trajectory determination process, the method achieves a higher level of precision and a more comprehensive visualization, catering to the critical need for accuracy and reliability in medical imaging processes.

Advantageously, said trajectories can be determined as the orbits of a given vector field, said vector field might be oriented from a first border defined by the second sub-parts to a second border defined by the third sub-parts, or oriented from the second border to the first border. For instance, said vector field may be defined as the gradient vector field of a scalar monotone function whose maximum is attained everywhere in the second border and whose minimum is attained everywhere in the first border. Consequently, the gradient of said function is a vector field pointing from the first border to the second border.

In a cumulative embodiment of the invention, for each first sub-part of said region of interest, said value of a predetermined local characteristic is computed from the length of a segment of said trajectory which encompasses said first sub-part of said region of interest, said segment being defined as the part of said trajectory strictly contained in said region of interest.

Advantageously, the method's provision for computing the value of a predetermined local characteristic from the length of a segment of the trajectory, which is strictly contained within the region of interest, introduces a high degree of precision and relevance in capturing the local characteristics of surrounding tissue features. By utilizing the length of the trajectory segment within the region of interest, the method can provide a more accurate quantification of local characteristics, as the thickness value of the region of interest or a characteristic derived from this thickness value, which are essential for a detailed and informative visualization. This approach ensures that the computations are directly related to the spatial and structural attributes of the surrounding tissue features, thereby enhancing the quality and informativeness of the visualizations generated. This meticulous computation can lead to a richer representation of the anatomical structures, providing more insightful information which could be pivotal for accurate diagnostics or effective treatment planning. Through such a precise and methodical computation, the method optimizes the extraction of meaningful information from medical images, significantly bolstering the utility and efficacy of the medical imaging process.

Advantageously, the explicit emphasis on computing the thickness value of the region of interest or a characteristic derived from this thickness value, like the density measured as cumulated thicknesses of fat-pads over the total length of the trajectory in instances of multiple layers, significantly enhances the method's capability to provide insightful and relevant data from the medical imagery. The nuanced approach of calculating segment length in straight trajectories and arclength in curved trajectories, by traversing the trajectory from the border to the dilated border and incrementing the length each time a pixel of the region of interest is traversed, lends a high degree of precision to the process. This precision is particularly crucial when capturing the complex anatomical structures and surrounding tissue features in medical imaging, ensuring that the visualization is both accurate and informative. The method's ability to derive critical characteristics like thickness and density from the trajectory segment contained within the Rol, while accommodating the trajectory's geometry, whether straight or curved, showcases a thoughtful and meticulous approach to extracting valuable information from medical imaging, which could be instrumental in making accurate medical assessments and decisions.

In a cumulative embodiment of the invention, for each first sub-part of said region of interest, said value of a predetermined local characteristic is computed from the length of a segment of said trajectory which encompasses said first sub-part of said region of interest, said segment extending from the second sub-part defining said trajectory to a first sub-part located both on said trajectory and on a border of the region of interest.

Advantageously, the method's approach of computing the value of a predetermined local characteristic from the length of a segment of the trajectory, which extends from the second sub-part defining the trajectory to a first sub-part located both on the trajectory and on the border of the region of interest, enables a precise and context-aware extraction of local characteristics. This meticulous approach ensures that the computed characteristics are highly relevant and accurately represent the spatial relationships between the tissue and the surrounding tissue features. The segment's defined extension, from the tissue surface to a border of the region of interest, ensures that the local characteristic computation is grounded on a logical and anatomically relevant basis, thereby enhancing the realism and the informative value of the resulting visualization. This defined segment of the trajectory provides a structured framework for computing local characteristics, which could lead to more accurate and informative visualizations, facilitating better diagnostic or therapeutic decisions. The method's precise and logical approach to determining the segment for characteristic computation aligns well with the essential requirement of accuracy and contextual relevance in medical imaging processes, making it a valuable advancement in the field.

Advantageously, in a particular case, the method's capability to compute distances to specific anatomical features like fat-pads, either to their inner or outer borders, stands as a significant asset in medical imaging. This precise distance computation, encompassing the thickness plus the distance to the fat-pad, provides a detailed insight into the anatomical structures and their spatial relationships, which is crucial for accurate diagnostics and treatment planning. In a variant concerning scar tissue, the method's approach of dilating from the endocardium towards the epicardium to establish the trajectory, and measuring the total distance, facilitates a thorough assessment of myocardial thickness. Additionally, the calculation of the ratio of endocardium-scar distance to endocardium-epicardium distance presents a nuanced metric that could be instrumental in evaluating the extent of scarring and its impact on myocardial function. This level of detailed analysis and the ability to derive meaningful metrics, like the ratio of distances concerning scar tissues, significantly augments the method's utility in providing a comprehensive understanding of the anatomical and pathological conditions, thereby enabling a more informed medical analysis and decision-making process. Through these nuanced computations and the provision for deriving insightful metrics, the method exemplifies a notable advancement in the realm of medical imaging, catering to the evolving demands of clinical diagnostics and therapeutic planning.

In a cumulative embodiment of the invention, wherein, said region of interest being composed of different disconnected layers of first sub-parts, for each first sub-part of said region of interest, said value of a predetermined local characteristic is computed from the number of layers traversed by said trajectory which encompasses said sub-part of said region of interest.

Advantageously, for instances where the region of interest is composed of different disconnected layers of first sub-parts, brings forth a nuanced approach to evaluating complex anatomical structures. By computing the value of a predetermined local characteristic based on the number of layers traversed by the trajectory encompassing a sub-part of the region of interest, a deeper level of understanding regarding the spatial and structural intricacies of the region is achieved. This approach allows for a thorough examination of the multilayered aspects of the region of interest, providing a more detailed and comprehensive visualization. The method's capability to accurately account for and represent the disconnected layers traversed by the trajectory adds a layer of precision and depth to the analysis, which could be pivotal in rendering accurate diagnostic assessments or therapeutic strategies. The enhanced granularity in understanding and visualizing the various layers within the region of interest significantly contributes to the utility and effectiveness of the medical imaging process, making it a valuable advancement in achieving nuanced and informative visualizations.

Advantageously, in a particular scenario where a fat-pad consists of several discontinuous but related layers of fat, the method's detailed approach shines in extracting meaningful insights from complex anatomical configurations. The computation of a predetermined local characteristic such as the cumulative thickness of all crossed layers, or statistical indicators like average, maximum, or minimum thickness, provides a nuanced understanding of the fat-pad's structure. This fine-grained analysis not only elucidates the morphological attributes of the fat-pad but also potentially unveils the relational dynamics among the discontinuous layers of fat. By aggregating or statistically analyzing the thicknesses of the layers traversed by the trajectory, a richer and more informative representation of the fat-pad's anatomy is achieved. This level of detailed analysis is instrumental in rendering a comprehensive understanding of the anatomical region, which could be pivotal in clinical assessments or therapeutic deliberations. The method's ability to cater to such complex anatomical scenarios by providing meaningful metrics accentuates its versatility and efficacy in medical imaging, thereby extending a significant advantage in deciphering intricate anatomical structures and aiding in more informed medical decision-making.

In a cumulative embodiment of the invention, for each first sub-part of said region of interest, said value of a predetermined local characteristic is computed from a statistical indicator of the intensities of the first sub-parts of the region of interest which are traversed by said trajectory which encompasses said first sub-part of said region of interest.

Advantageously, the method's provision for computing the value of a predetermined local characteristic based on a statistical indicator of the intensities of the first sub-parts of the region of interest traversed by the encompassing trajectory introduces a robust mechanism for capturing and quantifying the inherent properties of the region of interest. By utilizing statistical indicators, a more comprehensive understanding of the variations and distributions of intensity values within the traversed sub-parts is attained, which can be instrumental in achieving a nuanced visualization of the tissue and surrounding features. This statistical approach allows for a data-driven, objective assessment of the local characteristics, which could unveil critical insights into the anatomy or pathology present. The method's ability to harness the statistical attributes of intensity values within the region of interest, as defined by the trajectory, significantly augments the depth and the quality of information extracted from the medical imagery. This, in turn, potentially facilitates more accurate diagnostic evaluations or therapeutic interventions, as the visualizations generated would be reflective of a thorough and data-informed analysis of the underlying anatomical structures. Through such a refined computational approach, the method enhances the precision and the utility of medical imaging, catering to the critical need for accuracy and detailed analysis in clinical settings.

If desired, the statistical indicator utilized could vary to include the minimum intensity, maximum intensity, average intensity, among others, depending on the specific requirements of the analysis at hand. Each of these statistical indicators could provide a distinct perspective on the intensity distribution within the region of interest, thereby enabling a tailored approach to understanding the underlying anatomical or pathological conditions. For instance, the minimum or maximum intensity might highlight the extremes of intensity variation within the region of interest, potentially indicating areas of abnormal tissue characteristics or pathological alterations. On the other hand, the average intensity could provide a generalized understanding of the intensity distribution, offering a holistic view of the region's condition. This flexibility in choosing the statistical indicator aligns well with the method's objective of providing a detailed and nuanced visualization based on a data-driven analysis of the region of interest. It opens avenues for a more customized analysis, wherein the choice of the statistical indicator could be aligned with the diagnostic or therapeutic objectives, thereby significantly enhancing the method's applicability and effectiveness in varied medical imaging scenarios. Through such tailored analysis, the method exemplifies a high degree of adaptability and precision, catering to the evolving demands of medical imaging in clinical practice.

In a cumulative embodiment of the invention, the method comprises a further step of displaying said 3D model of said tissue, wherein each mesh element of said sub-mesh is displayed with a graphical feature depending on the value of the predetermined characteristic of its associated sub-part of the region of interest.

Advantageously, the inclusion of a further step for displaying the 3D model of the tissue, where each mesh element of the sub-mesh is depicted with a graphical feature based on the value of the predetermined characteristic of its associated sub-part of the region of interest, greatly enhances the visual interpretation and understanding of the analyzed data. This graphical representation facilitates a clear and intuitive visualization of the varying characteristics across different regions, thereby providing a more effective and user-friendly interface for medical practitioners to interpret the results. By correlating graphical features with the predetermined characteristics, a more direct and effective communication of the analysis results is achieved, potentially aiding in quicker and more accurate diagnostic or therapeutic decisions. This visually enriched representation of the 3D model not only makes the method more accessible and insightful but also leverages visual cognition to convey complex data in an easily digestible manner. This graphical embodiment of analyzed data significantly contributes to bridging the gap between complex data analysis and practical clinical interpretation, fostering a better understanding and utilization of medical imaging data in clinical settings.

In a cumulative embodiment of the invention, wherein each mesh element of said sub-mesh is displayed with a color depending on the value of the predetermined local characteristic of its associated sub-part of the region of interest.

Advantageously, the method's feature of displaying each mesh element of the sub-mesh with a color based on the value of the predetermined local characteristic of its associated sub-part of the region of interest significantly enhances the visual clarity and interpretability of the 3D model. By employing a color-coding scheme, complex data pertaining to the local characteristics of the tissue and surrounding features are translated into an intuitive, visually engaging representation. This color-based visualization provides a quick, at-a-glance understanding of the spatial distribution of the predetermined local characteristics, aiding in the rapid assimilation of crucial information. This, in turn, can potentially expedite the diagnostic or therapeutic decision-making process, offering immediate visual cues to the medical practitioners. Additionally, the use of color enhances the aesthetic and cognitive ease of the visualization, making the analysis results more accessible and easier to interpret. The color-coding approach essentially transforms the analytical findings into a visually coherent and easily digestible format, thereby bridging the technical complexities of medical imaging analysis with the practical necessities of clinical interpretation, and significantly contributing to improved medical imaging diagnostics and patient care.

Building on the visual enhancement facilitated by color-coding, the method's incorporation of a colormap, where the total range of potential values of the predetermined characteristic is decomposed into consecutive sub-ranges, each associated with a consecutive color of a color palette, further refines the visualization process. This systematic decomposition and association to a color palette ensure that each voxel is displayed with the color corresponding to the sub-range to which its value belongs, thereby providing a detailed, graduated visual representation of the data. This graduated color scheme facilitates a nuanced understanding of the variations in the predetermined local characteristics across the region of interest, enabling a more precise interpretation of the data. It allows for an immediate visual differentiation and understanding of the relative values of the predetermined characteristic across different sub-parts of the region of interest. This colormap approach, thus, not only enhances the visual appeal of the 3D model but also significantly contributes to a more informed and accurate interpretation of the medical imaging data. By rendering complex data into a visually intuitive and graduated color-coded format, the method effectively bridges the technical data analysis with practical, user-friendly visualization, enhancing the ease and accuracy of clinical interpretations.

In a cumulative embodiment of the invention, the method comprises a further step of generating a supplementary mesh from said sub-mesh, each mesh element of said supplementary mesh being extruded outwardly from a mesh element of said sub-mesh and being labelled with the same value of the predetermined local characteristic than this mesh element of said sub-mesh.

Advantageously, the method's incorporation of a step to generate a supplementary mesh from the sub-mesh, with each mesh element of the supplementary mesh being extruded outwardly from a mesh element of the sub-mesh and labeled with the same value of the predetermined local characteristic, significantly enhances the three-dimensional representation of the tissue and the associated region of interest. This extrusion process creates a more pronounced, tangible visualization of the predetermined local characteristics, making it easier to discern the spatial and structural nuances of the region of interest. By extending the geometry of the sub-mesh into a supplementary mesh while preserving the labeling of predetermined local characteristics, a richer, more informative three-dimensional model is constructed. This not only augments the visual clarity and understanding of the medical imaging data but also potentially facilitates a more intuitive interaction with the model, aiding in better diagnostic or therapeutic evaluations. The method's ability to provide a more detailed, extruded representation while maintaining the integrity of the data labeling significantly contributes to delivering a more effective and user-friendly visualization, thereby potentially enhancing the accuracy and efficiency of clinical assessments and interventions.

In a variant of the method, the creation of an additional mesh through extrusion adds a further dimension to the visualization process. This extrusion is performed by extending the geometry of each mesh element of the sub-mesh outwardly to generate corresponding elements of the additional mesh. The extrusion process essentially takes each mesh element of the sub-mesh and projects it outwardly, creating a new set of mesh elements that form the additional mesh. This additional mesh, generated through extrusion, inherits the labeling of the predetermined local characteristics from the original sub-mesh elements, ensuring that the data integrity and the correlation between the mesh geometry and the underlying medical data are preserved. The extrusion not only enhances the three-dimensional representation of the tissue and the region of interest but also provides a more pronounced visualization of the predetermined local characteristics. By offering a method to create an additional mesh through extrusion, the visualization process becomes more versatile and the resulting three-dimensional model becomes more informative. This added capability to generate an extruded additional mesh enriches the visual representation and provides a more nuanced understanding of the anatomical or pathological conditions being investigated, thereby furthering the method's utility in medical imaging analysis and clinical diagnostics.

The subject of the invention is also a computing device for the implementation of the method according to one of the embodiments, said computing device comprising a memory arranged to receive at least one mapping of points and
Figure 1 is a logic flow diagram that depicts a computer-implemented method, according to an embodiment of the invention;
Figure 2 shows a schematic representation of a 3D model comprising a mesh and representing a tissue of a patient's heart;
Figure 3 shows a schematic representation of an image of a tissue of said patient's heart and a surrounding tissue feature.
Figure 4 shows a schematic representation of the result of a segmentation step of the method depicted in Figure 1 when implemented on the image of a tissue of said patient's heart and surrounding tissue features extracted from said image of Figure 3.
Figure 5 shows a schematic representation of an image comprising a tissue of a patient's heart and a plurality of first sub-parts inside a region of interest.
Figure 6 shows a schematic representation of the extraction step which comprises, for a plurality of second sub-parts of said image corresponding to the surface of said tissue; determining a trajectory encompassing said second sub-part and at least one first sub-part of said region of interest.

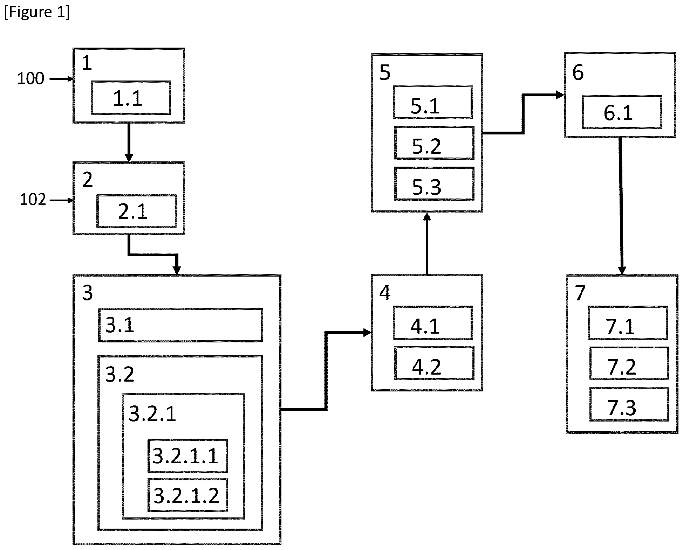

Reference is made to Figure 1 which shows a computer-implemented method for the visualization of surrounding tissue features located out of a surface of the tissue of a patient's heart 51.

The method starts with step 1 of receiving a 3D model 100 of the tissue 50 of the patient's heart 51. Step 1 comprises a sub-step 1.1 of determining that said 3D model 100 comprises a mesh 101 that represents the surface of the heart tissue 50.

Step 1 is followed by a step 2 of reception receives at least one image 102. Step 2 comprises a sub-step 2.1 of identifying that said image not only represents the heart tissue 50 but also showcases the surrounding tissue feature 52 which is located outside the surface of the heart tissue 50.

In step 3, the method focuses on segmenting the acquired image 102. Step 3 comprises a sub-step 3.1 of identifying at least one region of interest 103 that corresponds to the surrounding tissue feature 52 of the heart. Said region of interest 103 comprising a plurality of first sub-parts 104. Furthermore, step 3 comprises another sub-step 3.2 of selection of specific sub-parts of the image 102 that are outside the heart tissue 50. Said sub-step 3.2 comprises a further sub-step 3.2.1 of evaluation of the intensity and the distance of said sub-parts, according to a sub-step 3.2.1.1 of selection of sub-parts whose intensity is below a predetermined threshold, and a sub-step 3.2.1.2 of selection of sub-parts whose proximity to the heart tissue is less than a predetermined distance threshold.

Step 4 involves the extraction of specific information from the image 102. Step 4 comprises a sub-step 4.1 of extracting, for each of these identified first sub-parts 104 in the region of interest 103, at least one value 105 which represents a predetermined local characteristic of the said first sub-part 104. Moreover, step 4 comprises another sub-step 4.2 wherein for multiple second sub-parts 108 of the image 102 associated with the heart tissue surface, a trajectory is charted. Said sub-step 4.2 comprises a further sub-step 4.2.1 of defining the trajectory, which encompasses both the second sub-part 108 and at least one first sub-part 104 of the region of interest. The value of said local characteristic is dependent on the trajectory 53 that encompasses it.

Step 5 emphasizes the association of the first sub-parts 104 with the mesh 101 of the 3D model 100. Step 5 comprises a sub-step 5.1 of associating each of these first sub-parts 104 with a particular mesh element based on their respective positions. As a result of said association, step 5 comprises another sub-step 5.2 where these collectively associated mesh elements form a sub-mesh 106. Further, step 5 also comprises a sub-step 5.3 of labelling each element of said sub-mesh 106 with the value 107 obtained from its associated first sub-part from the region of interest.

In step 6, the 3D model 100 of the heart tissue 50 is displayed. Step 6 comprises a sub-step 6.1 of displaying each mesh element of the sub-mesh 106 with a feature based on the value of its associated sub-part 104 from the region of interest.

Lastly, step 7 is about generating a supplementary mesh 110 from the previously formed sub-mesh 106. Step 7 comprises a sub-step 7.1 of creating said supplementary mesh 110 from the sub-mesh 106. Following this, step 7 comprises another sub-step 7.2 of extruding each mesh element of said supplementary mesh 110 outwardly from its counterpart in the sub-mesh 106. Finally, step 7 comprises a sub-step 7.3 of labelling each element of said supplementary mesh 110 with the same value 105 as its corresponding element in the sub-mesh 106.

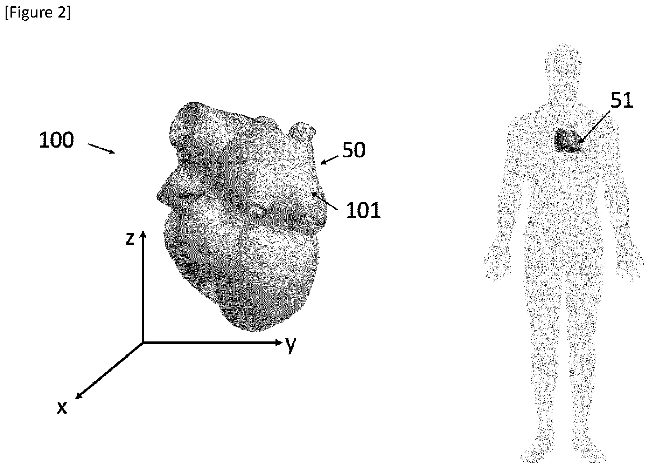

Figure 2 displays a 3D model, denoted by reference 100, which includes a mesh 101. This 3D model 100 represents the tissue 50 of a patient's heart 51, and the mesh 101 captures the intricate surface of the heart tissue, showcasing individual elements that constitute the mesh 101.

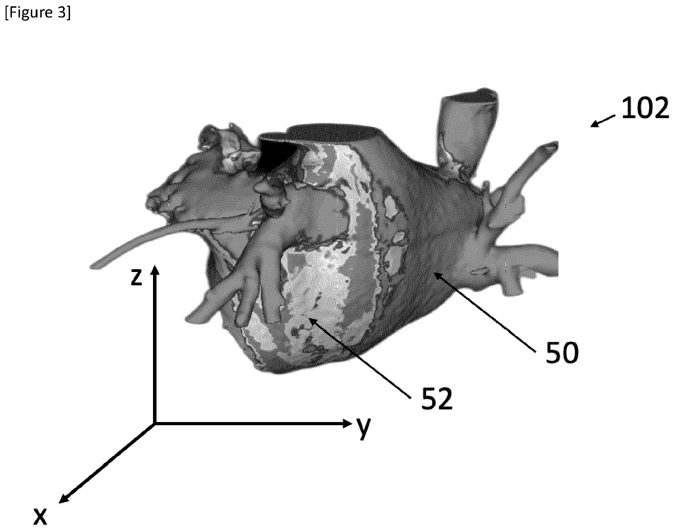

Figure 3 offers an image, assigned the reference 102, capturing the tissue of the patient's heart, labelled 50, alongside a surrounding tissue feature, identified as 52. The image provides a visual of both the heart tissue and the external tissue feature that lies beyond the surface of the heart tissue.

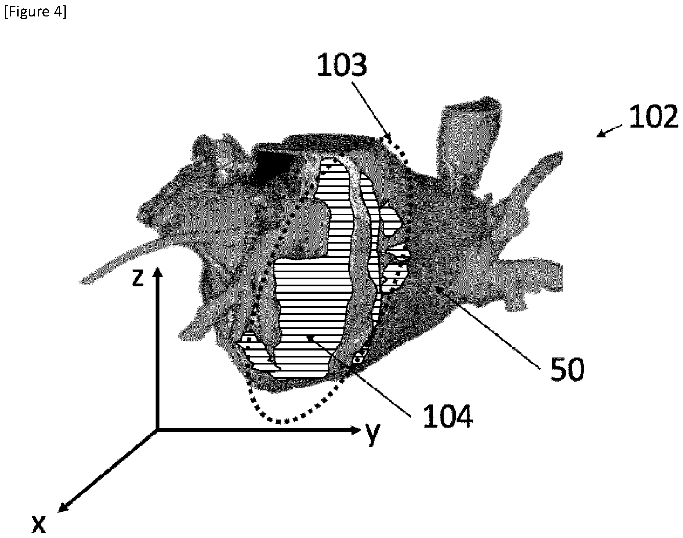

Figure 4 provides a schematic representation that specifically showcases the results of the segmentation step applied to the image 102, which depicts the tissue of a patient's heart, referenced as 50. Within this segmented image, there is a clearly delineated region of interest 103. This region is particularly significant as it identifies areas within the heart tissue 50 that are of keen interest. Within the boundaries of the region of interest 103, numerous first sub-parts can be observed, each denoted as 104.

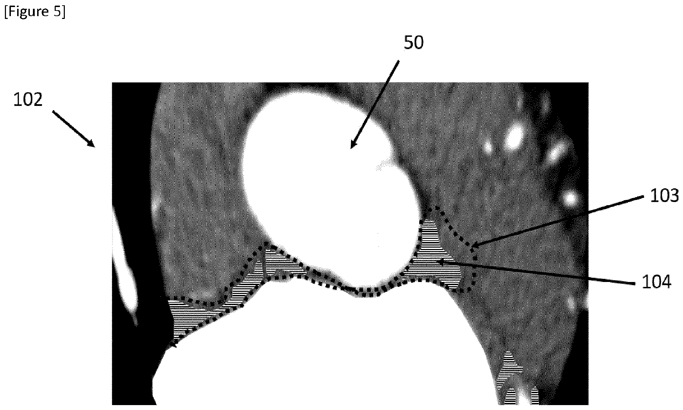

The representation in Figure 4 underscores the relationship between the original image 102 and the segmented elements, including the region of interest 103 and the associated first sub-parts 104. It provides a comprehensive view of how the original image 102 undergoes segmentation to spotlight specific areas and features of the heart tissue 50 that are pivotal for subsequent stages of the visualization method. Figure 5 presents a schematic representation of the image, denoted as 102, which encompasses the tissue of a patient's heart, designated as 50. Within this image, a discernible region of interest, highlighted by reference 103, can be observed. This region of interest is derived from the overarching image, serving as a focal point for the method's operations. Inside this region, multiple first sub-parts, signified by 104, can be identified. These first sub-parts are integral components, representing distinct areas or features within the heart tissue and its immediate surroundings. They are crucial for the ensuing steps of the method, offering a structured approach to understanding and visualizing the heart tissue in its entirety.

Furthermore, the illustration in Figure 5 provides clarity on the relationship between the heart tissue and these first sub-parts. The positioning, size, and distribution of the first sub-parts within the region of interest offers insights into their significance and role in the overall visualization process. By focusing on these elements within the image 102, the method is better equipped to extract, analyze, and eventually display crucial information about the heart tissue 50 and its associated features.

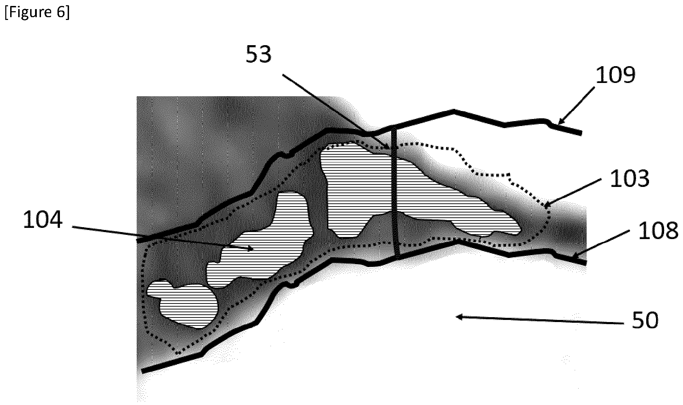

Figure 6 offers a detailed schematic focusing on the extraction process, primarily revolving around the trajectories. Highlighted within are the second sub-parts of the image, denoted by reference 108, which correlate with the surface of the heart tissue, labelled 50. For each of these second sub-parts, a trajectory, represented by 53, is meticulously drawn. This trajectory is not just a mere connection, but a comprehensive path that spans between the specific second sub-part, 108, and at least one associated first sub-part, 104, located within the predefined region of interest, 103. It's through these trajectories, 53, that the relationship and spatial orientation between the second sub-parts and the first sub-parts are visually and analytically established. The trajectory is instrumental in determining the thickness of the surrounding tissue feature, as it measures the distance between each second sub-part and its corresponding first sub-part. This thickness, governed by the length and direction of the trajectory, provides critical insight into the structural depth and arrangement of the tissue, aiding in a more nuanced understanding. Furthermore, Figure 6 integrates this trajectory-based information with the mesh elements, referenced by 109, elucidating how they collectively offer a thorough depiction of the heart tissue's surface and its immediate surroundings.

The methods disclosed herein may also be implemented by software programs executable by a computer system. Further, implementations may include distributed processing and parallel processing, especially for processing in parallel several or all data in the data sets.

The illustrations described herein are intended to provide a general understanding of the structure of various embodiments. These illustrations are not intended to serve as a complete description of all the elements and features of apparatus, processors and systems that utilizes the structures or methods described therein. Many other embodiments or combinations thereof may be apparent to those of ordinary skills in the art upon reviewing the disclosure by combining the disclosed embodiments. Other embodiments may be utilized and derived from the disclosure, such that structural and logical substitutions and changes may be made without departing from the scope of the disclosure.

Further, the disclosure and the illustrations are to be considered as illustrative rather than restrictive, and the appended claims are intended to cover all such modifications, enhancements, and other embodiments, which fall within the true spirit and scope of the description. Thus, the scope of the following claims is to be determined by the broadest permissible interpretation of the claims and their equivalents and shall not be restricted or limited by the foregoing description.

## Claims

1. A computer-implemented method for the visualization of surrounding tissue features located out of a surface of a tissue of a patient's organ (51), the method comprising:
a. step (1) of receiving a 3D model (100) of a tissue (50) of said patient's organ (51), said 3D model (100) comprising a mesh (101) representing a surface of said tissue (50) of said patient's organ (51);
b. step (2) of receiving at least one image (102) representing said tissue (50) and at least one surrounding tissue feature (52) located out of said surface of said tissue (50);
c. step (3) of segmenting, in said image (102), at least one region of interest (103) corresponding to said surrounding tissue feature (52), said region of interest (103) being formed by a plurality of first sub-parts (104);
d. step (4) of extracting, from said image (102) and for each first sub-part (104) of said region of interest (103), at least one value (105) of a predetermined local characteristic of said first sub-part (104) of said region of interest (103);
e. a step (5) of associating each first sub-part (104) of said region of interest (103) to at least one mesh element of said mesh (101) of the 3D model (100), based on said first sub-part (104) and mesh element's positions; wherein said associated mesh elements form together a sub-mesh (106) of said mesh (101); each mesh element of said sub-mesh (106) being labelled with the value (107) of the predetermined local characteristic of its associated first sub-part of said region of interest.

2. The method according to claim 1, wherein said segmentation step (3) comprises the selection of any sub-part (104) of said image (102) which is located outside of said tissue, whose distance to said tissue is lower than a predetermined threshold.

3. The method according to one of the previous claims, wherein said extraction step (4) comprises, for a plurality of second sub-parts (108) of said image (102) corresponding to said surface of said tissue (50); determining a trajectory encompassing said second sub-part (108) and at least one first sub-part (104) of said region of interest (103); and wherein, for each first sub-part (104) of said region of interest (103), said value of a predetermined local characteristic is computed depending on said trajectory (53) which encompasses said first sub-part (104) of said region of interest (108).

4. The method according to the previous claim, wherein each trajectory (53) is a trajectory encompassing a second sub-part (108) corresponding to said surface of said tissue (50) and at least one fist sub-part (104) of said region of interest (103) with a direction normal to said surface at the location of said second sub-part (108).

5. The method according to claim 3, wherein said trajectories (53) are determined with:
a. dilating the plurality of second sub-parts (108) to define a plurality of third sub-parts (109) which englobes said region of interest (103),
b. determine a plurality of trajectories (53) each joining one second sub-part (108) to one third sub-part (109).

6. The method according to one of the claims 3 to 5, wherein, for each first sub-part (104) of said region of interest (103), said value of a predetermined local characteristic is computed from the length of a segment of said trajectory (53) which encompasses said first sub-part (104) of said region of interest (103), said segment being defined as the part of said trajectory (53) strictly contained in said region of interest (103).

7. The method according to one of the claims 3 to 6, wherein, for each first sub-part (104) of said region of interest (103), said value of a predetermined local characteristic is computed from the length of a segment of said trajectory (53) which encompasses said first sub-part (104) of said region of interest (103), said segment extending from the second sub-part (108) defining said trajectory (53) to a first sub-part (104) located both on said trajectory (53) and on a border of the region of interest (103).

8. The method according to one of the claims 3 to 7, wherein, said region of interest (103) being composed of different disconnected layers of first sub-parts (104), for each first sub-part (104) of said region of interest (103), said value (105) of a predetermined local characteristic is computed from the number of layers traversed by said trajectory (53) which encompasses said sub-part (104) of said region of interest (103).

9. The method according to one of the claims 3 to 8, wherein, for each first sub-part (104) of said region of interest (103), said value (105) of a predetermined local characteristic is computed from a statistical indicator of the intensities of the first sub part (104) of the region of interest (103) which are traversed by said trajectory (53) which encompasses said first sub-part (104) of said region of interest (103).

10. The method according to one of the previous claims, characterized it comprises a further step (6) of displaying said 3D model (100) of said tissue (50), wherein each mesh element of said sub-mesh (106) is displayed with a graphical feature depending on the value of the predetermined characteristic of its associated sub-part (104) of the region of interest (103).

11. The method according to the previous claim, wherein each mesh element of said sub-mesh (106) is displayed with a color depending on the value (105) of the predetermined local characteristic of its associated sub-part (104) of the region of interest (103).

12. The method according to one of the claims 1 to 9, characterized it comprises a further step (6.1) of generating a supplementary mesh (110) from said sub-mesh (106), each mesh element of said supplementary mesh (110) being extruded outwardly from a mesh element of said sub-mesh (106) and being labelled with the same value (105) of the predetermined local characteristic than said mesh element of said sub-mesh (106).
